# EUROPÄISCHE PATENTSCHRIFT

(11) **EP 0 584 641 B1**
(45) Veröffentlichungstag und Bekanntmachung des Hinweises auf die Patenterteilung: **03.02.1999**
(21) Anmeldenummer: 93112816.9
(22) Anmeldetag: 10.08.1993
(51) Int. Cl.: B05B 17/06, B05B 5/00

(54) **Verfahren und Vorrichtung zur Erzeugung von Nebeln**
Method and device for producing mists
Procédé et dispositif de production de brouillard

(30) Priorität: 25.08.1992 DE 4228229
(43) Veröffentlichungstag der Anmeldung: 02.03.1994
(73) Patentinhaber: Jossner, Dieter, 77866 Rheinau (DE)
(72) Erfinder: Jossner, Dieter, 77866 Rheinau (DE)
(74) Vertreter: Sajda, Wolf E., Dipl.-Phys.

(56) Entgegenhaltungen:
- DE-A- 3 516 144
- DE-C- 885 916
- US-A- 3 815 829
- PATENT ABSTRACTS OF JAPAN vol. 5, no. 16 (C-041)30. Januar 1981 & JP-A-55 144 090 (KOMATSU YOSHIHISA) 10. November 1980

## Beschreibung

Die Erfindung betrifft ein Verfahren und eine Vorrichtung zur Erzeugung von Nebeln gemäß dem Oberbegriff der Ansprüche 1 und 14, mit denen aus einer Flüssigkeit feinstverteilte Flüssigkeitströpfchen erzeugt und anschließend einem zu behandelnden Objekt zugeführt werden. Ein derartiges Verfahren und eine entsprechende Vorrichtung ist aus DE-A-3516144 bekannt.

Strahlen aus solchen feinstverteilten Flüssigkeitströpfchen werden beispielsweise für kosmetische und therapeutische Zwecke verwendet, um beispielsweise die Haut eines Patienten oder beliebige Substrate zu behandeln. Herkömmlicherweise verwendet man für diese Zwecke ein Gefäß, in welchem Wasser zum Kochen gebracht wird. Der dabei entstehende Wasserdampf wird dann über eine Leitung dem zu behandelnden Objekt zugeführt. Eine Anordnung oder ein Verfahren dieser herkömmlichen Art bringen aber eine Vielzahl von Nachteilen und Gefahren mit sich.

Heißer Wasserdampf ist häufig nicht zu sehen, so daß sich bei unvorsichtigem Vorgehen schwere Verbrühungen ereignen können. Wenn der Flüssigkeit, beispielsweise Wasser, biologische Substanzen beigefügt sind, werden diese häufig durch das Kochen zerstört. Ein weiterer Nachteil besteht darin, daß Geräte dieser Bauart eine hohe Leistungsaufnahme haben, die meistens mehr als 2 kW beträgt. Außerdem vergeht üblicherweise eine Wartezeit von 5 bis 10 Minuten, bis das Gerät betriebsbereit ist und Wasserdampf liefert. Nach der Benutzung des Gerätes muß das Gerät abkühlen können, damit die heiße Vorrichtung keinen Schaden anrichten kann.

Aus der DE 37 08 933 A1 ist ein Verfahren mit den dazu gehörigen Vorrichtungen zur Dosierung feiner und fester Werkstoff- und Wirkstoffteilchen mit Hilfe schwingungserzeugender Systeme bekannt. Dort ist erläutert, daß das Dosieren fester Werkstoff/Wirkstoffpartikel derzeit ein großes Problem ist, wenn auf die Verwendung von Flußmitteln und Treibgasen verzichtet werden muß. Der Grund ist der, daß die Düse zum Aufbringen solcher Teilchen häufig verstopft, was durch die Oberflächenrauhigkeit der Teilchen hervorgerufen wird. Aus diesem Grunde werden dort schwingungserzeugende Systeme verwendet, welche die Teilchen in Bewegung setzen und damit das Verstopfen von Düsenöffnungen verhindern. Weiterhin soll dabei durch eine Änderung der Frequenz und der Amplitude die Austrittsmenge der Teilchen pro Zeiteinheit frei variiert werden. Die Problematik der Erzeugung von Nebeln, mit denen aus einer Flüssigkeit feinstverteilte Flüssigkeitströpfchen erzeugt und anschließend einem zu behandelnden Objekt zugeführt werden, ist in dieser Druckschrift nicht behandelt.

Der Erfindung liegt daher die Aufgabe zugrunde, ein Verfahren und eine Vorrichtung der eingangs genannten Art anzugeben, bei denen die Klumpen- und Clusterbildung weitgehend verringert wird und die Flüssigkeitströpfchen ein verbessertes Schwebeverhalten zeigen.

Die erfindungsgemäße Lösung besteht darin, ein Verfahren der eingangs genannten Art so durchzuführen, daß die Flüssigkeitströpfchen in der Flüssigkeitströpfchen-Strömung mit einem amplitudenmodulierten Magnetfeld magnetisiert und/oder mit amplitudenmoduliertem Licht bestrahlt werden, bevor sie anschließend zur Behandlung eines Objektes emittiert werden.

Die erfindungsgemäße Vorrichtung zur Erzeugung von Nebeln umfaßt ein Zerstäubergefäß mit einem Behälter zur Aufnahme einer Flüssigkeit, eine Einrichtung, die der Flüssigkeit Energie zuführt, um aus der Flüssigkeit feinstverteilte Flüssigkeitströpfchen zu erzeugen, und ein Gebläse, mit dem eine Strömung von Flüssigkeitströpfchen ausgebildet und zum Auslaß der Vorrichtung bewegt wird, von dem die Flüssigkeitströpfchen einem zu behandelnden Objekt zugeführt werden, wobei der Behälter zur Aufnahme der Flüssigkeit einen Schwingquarz aufweist, der an einen Oszillator angeschlossen ist und ist dadurch gekennzeichnet, daß das Zerstäubergefäß einen Kanal aufweist, der von einer Magnetspule umgeben ist, mit der ein amplitudenmoduliertes Magnetfeld erzeugbar ist, und/oder eine Strahlungsquelle besitzt, mit der amplitudenmoduliertes Licht erzeugbar ist, mit dem die Flüssigkeitströpfchen bestrahlt und angeregt werden, bevor sie aus dem Auslaß der Vorrichtung emittiert werden.

Vorteilhafte Weiterbildungen des Verfahrens und der Vorrichtung gemäß der Erfindung sind in den Unteransprüchen angegeben.

Mit dem Verfahren und der Vorrichtung gemäß der Erfindung wird die Aufgabe in zufriedenstellender Weise gelöst. Insbesondere ist eine gefahrlose Anwendung der damit erzeugten Strahlen von Flüssigkeitströpfchen möglich, da Verbrühungen ausgeschlossen sind.

Da die Flüssigkeit nicht gekocht zu werden braucht, um die Flüssigkeitströpfchen zu erzeugen, entfällt die lange Wartezeit bis zur Betriebsbereitschaft. Dies gilt unabhängig davon, ob im einfachsten Falle Wasser, gegebenenfalls sterilisiertes Wasser, oder eine beliebige Trägerflüssigkeit mit einer zusätzlichen Wirksubstanz verwendet wird, bei der es sich um aromatische Öle, ätherische Öle, homöopathische Medikamente und/oder biologische Substanzen handeln kann. Derartige Wirksubstanzen werden in vorteilhafter Weise keinesfalls beeinträchtigt oder gar zerstört. Ein weiterer Vorteil besteht darin, daß gemäß der Erfindung ein weitaus geringerer Energieverbrauch zu verzeichnen ist, der in der Größenordnung von weniger als 100 W liegt und damit um einen Faktor von mehr als 20 unter dem Verbrauch von herkömmlichen Geräten liegt.

Die Erfindung wird nachstehend, auch hinsichtlich weIterer Merkmale und Vorteile, anhand der Beschreibung von Ausführungsbeispielen und unter Bezugnahme auf die beiliegenden Zeichnungen näher erläutert. Die Zeichnungen zeigen in
- Fig. 1: eine schematische Seitenansicht im Schnitt der Zerstäubereinrichtung der erfindungsgemäßen Vorrichtung; und in
- Fig. 2: eine schematische Seitenansicht im Schnitt der an die Zerstäubereinrichtung anschließenden Auslaßeinrichtung.

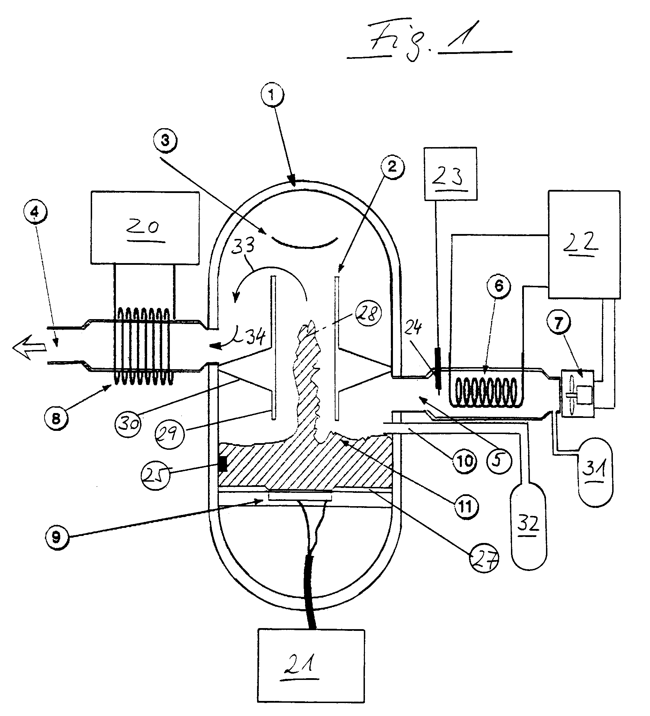

In Fig. 1 erkennt man ein Zerstäubergefäß 1, das in seinem unteren Bereich einen Behälter 27 zur Aufnahme einer Flüssigkeit 11 aufweist, aus der feinstverteilte Flüssigkeitströpfchen erzeugt werden sollen. Eine entsprechende Flüssigkeit wird über eine Leitung 10 von einer schematisch angedeuteten Flüssigkeitsversorgungsquelle mit einem Flüssigkeitsbehälter 32 zugeführt. Im Bereich des Behälters 27 ist zu diesem Zweck ein schematisch angedeuteter Pegeldetektor 25 angeordnet, der an die Flüssigkeitsversorgungsquelle angeschlossen ist und ein entsprechendes Signal liefert, falls ein Minimumpegel unterschritten wird, so daß die Flüssigkeit in Abhängigkeit vom Flüssigkeitsverbrauch automatisch nachfüllbar ist.

Innerhalb des Zerstäubergefäßes 1 ist eine Trenneinrichtung 2 vorgesehen, die dafür sorgt, daß nur feinstverteilte Flüssigkeitströpfchen aus dem Zerstäubergefäß 1 durch einen Kanal 4 austreten können. Diese Trenneinrichtung 2 weist eine quer verlaufende Trennwand 30 sowie einen durch die Trennwand 30 hindurchgehenden rohrförmigen Bereich 29 auf. Weiterhin ist oberhalb des rohrförmigen Bereiches 29 ein plattenförmiger Tropfschutz 3 angeordnet, der zweckmäßigerweise konvex in Richtung des rohrförmigen Bereiches 29 gekrümmt ist und für große Flüssigkeitstropfen eine Sperre bildet, die dann wieder nach unten in den Vorrat an Flüssigkeit 11 fallen. Die Trennwand 30 kann ferner nicht dargestellte Öffnungen aufweisen, durch die von der Oberseite des Zerstäubergefäßes 1 herunterfallende Tropfen wieder in den Vorrat an Flüssigkeit 11 gelangen.

Unterhalb der Trennwand 30 mündet ein Zuführungsbereich 5 für eine Gasströmung, die beispielsweise von einer schematisch angedeuteten Gasversorgung 31 geliefert wird. Im einfachsten Falle handelt es sich dabei um Luft der Atmosphäre, jedoch kann für spezielle Anwendungen auch ein spezielles Gas verwendet werden, beispielsweise Sauerstoff, Stickstoff oder ein Inertgas, wobei diese Gasströmung durch ein nicht dargestelltes Filter zugeführt werden kann.

Dem Zuführungsbereich 5 ist ein Gebläse 7 zugeordnet, das an eine Stromversorgung 22 angeschlossen ist, derart, daß die Geschwindigkeit bzw. der Durchsatz der Gasströmung aus diesem Zuführungsbereich 5 einstellbar sind. Weiterhin ist in diesem Zuführungsbereich 5 eine mit einer Wendel angedeutete Heizeinrichtung 6 vorgesehen, die ebenfalls an die Stromversorgung 22 angeschlossen ist. Unter Verwendung eines nicht dargestellten Temperaturmeßfühlers kann mit dieser Stromversorgung 22 die Temperatur der zugeführten Gasströmung auf einen geeigneten Wert eingestellt werden. Zusätzlich kann eine nicht dargestellte Kühleinrichtung in diesem Zuführungsbereich 5 vorgesehen sein, um auf diese Weise die zugeführte Gasströmung noch genauer auf die gewünschte Temperatur zu regeln. Für die Einstellung der Heizeinrichtung können beispielsweise NTC-Sensoren eingesetzt werden, während für die Kühleinrichtung beispielsweise Peltier-Elemente eingesetzt werden können.

Im Bodenbereich des Behälters 27, der zur Aufnahme der jeweiligen Flüssigkeit 11 gegebenenfalls auswechselbar ist, ist ein Schwingquarz 9 in dem Zerstäubergefäß 1 angeordnet. Bei diesem Schwingquarz handelt es sich zweckmäßigerweise um einen Piezokristall-Wandler, insbesondere einen PXE-Wandler, der an einen Oszillator in Form eines HF-Generators 21 angeschlossen ist.

In diesem Zusammenhang ist wichtig, daß der Oszillator genau auf der Resonanzfrequenz des scheibenförmigen Wandlers 9 schwingt. Diese Resonanzfrequenz ist abgesehen von den physikalischen Abmessungen des Wandlers selbst von dem Flüssigkeitspegel abhängig, der über diesem Wandler steht. Durch die automatische Zuführung von Flüssigkeit unter Verwendung des Pegeldetektors 25 wird dafür gesorgt, daß die Anordnung ständig auf der Resonanzfrequenz schwingen kann, wobei die mittlere Resonanzfrequenz bei etwa 1,6 MHz liegen kann.

Durch den Betrieb dieses Schwingquarzes bzw. Wandlers 9 mit dem HF-Generator 21 wird über dem Flüssigkeitsvorrat eine in Fig. 1 schematisch angedeutete Fontäne der Flüssigkeit 11 gebildet, aus der eine Wolke 28 von feinverteilten Flüssigkeitströpfchen gebildet wird. Die Austrittsgeschwindigkeit dieser Flüssigkeitströpfchen wird bestimmt durch die Gasströmung, die aus dem Zuführungsbereich 5 zugeführt und durch den rohrförmigen Bereich 29 hindurch geführt wird. Die Trennwand 30 bildet insofern eine wirksame Unterteilung des Zerstäubergefäßes 1. Auf diese Weise findet eine "kalte" Zerstäubung der Flüssigkeit unter Bildung von feinstverteilten Flüssigkeitströpfchen statt, die nach Umlenkung an dem Tropfschutz 3 in einen schematisch angedeuteten Kanal 4 gelangen, wie es mit Pfeilen 33 und 34 in Fig. 1 schematisch angedeutet ist.

Man erkennt, daß dieser Kanal 4 von einer Magnetspule 8 umgeben ist, die ihrerseits an eine Stromversorgung 20 angeschlossen ist. Diese Stromversorgung 20 liefert einen Strom, der mit einem breitbandigen Rauschspektrum amplitudenmoduliert ist, um mit dem dadurch erzeugten Magnetfeld die hindurchströmenden Flüssigkeitströpfchen magnetisch anzuregen. Dabei wird dem Umstand Rechnung getragen, daß diese Flüssigkeitströpfchen völlig verschiedene Teilchengrößen haben oder auch aus Clustern bestehen können, bei denen mehrere Flüssigkeitströpfchen aneinander haften. Zu diesem Zweck erzeugt die Stromversorgung 20 einen Strom, der amplitudenmoduliert ist mit einem Rauschspektrum in einem Bereich von etwa 1 Hz bis einigen hundert kHz, wobei üblicherweise ein oberer Wert von etwa 200 kHz genügt.

Zur Erzeugung eines derartigen Rauschspektrums kann beispielsweise ein mehrfach rückgekoppeltes Schieberegister verwendet werden, um die gewünschte Bandbreiten- und Amplitudenkonstanz zu erzielen. Alternativ können zur Erzeugung des Rauschspektrums auch Rauschdioden oder verpolte Transistoren verwendet werden. Die erforderliche Stromstärke in der Magnetspule 8 kann wie bei einem Audioverstärker mit einem Verstärker geliefert werden, wobei das erzeugte Magnetfeld dann das gleiche Rauschen aufweist wie der Rauschgenerator selbst.

Auf diese Weise erfolgt durch die Wirkung des Magnetfeldes eine zusätzliche Anregung der hindurchströmenden Flüssigkeitströpfchen, mit der Folge, daß diese zusätzliche Energie aufnehmen und schwingen. Die Erfahrung hat gezeigt, daß dadurch die Klumpen- oder Clusterbildung weitgehend verringert wird und die so behandelten Flüssigkeitströpfchen ein verbessertes, nämlich länger andauerndes Schwebeverhalten zeigen.

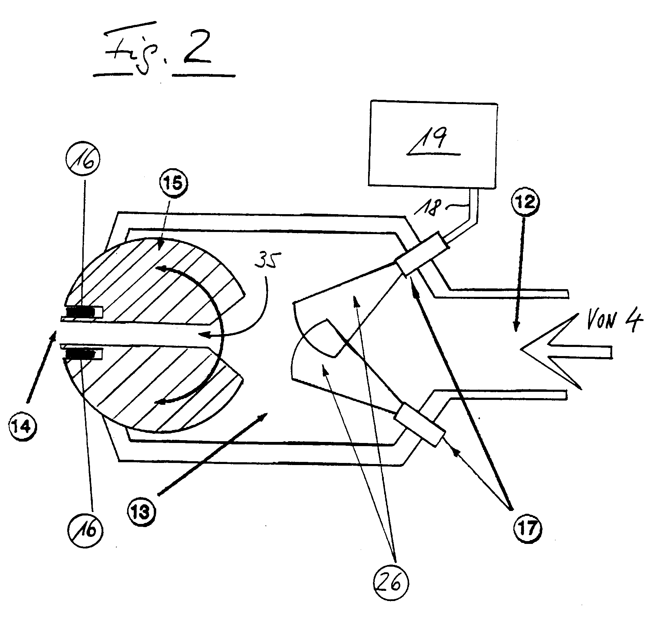

Wie aus Fig. 2 ersichtlich, geht der Kanal 4 mit einem Übergangsbereich 12 in einen Homogenisierungsbereich 13 über, der zur Vergleichmäßigung der Strömung von Flüssigkeitströpfchen dient. Im Auslaßbereich ist ein drehbares Auslaßteil 15 mit einem hindurchgehenden Auslaßkanal 35 vorgesehen, der in den eigentlichen Auslaß 14 mündet. Dieses drehbare Auslaßteil kann in Form einer Scheibe oder einer Kugel ausgebildet sein und dient dazu, die Strömung von Flüssigkeitströpfchen in der gewünschten Weise auszurichten.

In dem Homogenisierungsbereich 13 erkennt man ferner Strahlungsquellen 17, die in schematisch angedeuteter Weise über Leitungen 18 an eine Stromversorgung 19 angeschlossen sind. Insbesondere kann es sich bei diesen Strahlungsquellen 17 um Laserdioden handeln, wobei eine solche Strahlungsquelle 17 genügt, jedoch auch mehrere gleichmäßig um die Achse des Homogenisierungsbereiches 13 verteilte Strahlungsquellen 17 angeordnet sein können, um deren Wirkung zu erhöhen.

Jede dieser Strahlungsquellen 17 wird von einer spannungsgesteuerten Stromquelle 19 gespeist, wobei dem Strom für die Strahlungsquellen 17, insbesondere in Form von Laserdioden das gleiche Rauschspektrum aufgeprägt wird wie es auch für die Magnetspule 8 verwendet wird, also mit einem Rauschspektrum, das in einem Bereich von etwa 1 Hz bis einigen hundert kHz liegt, wobei auch hier ein oberer Wert von etwa 200 kHz ausreichend ist. Besonders zweckmäßig ist es, wenn zu diesem Zweck derselbe Rauschgenerator verwendet wird, um die Magnetspule 8 sowie die jeweiligen Strahlungsquellen 17 zu versorgen. Wenn nämlich die Magnetspule 8 und die jeweiligen Strahlungsquellen 17 an denselben Rauschgenerator angeschlossen sind, wird eine phasenstarre Kopplung des Magnetfeldes und der Photonen von dem Laser erreicht.

Von diesen Strahlungsquellen 17 wird Licht in Form von schematisch angedeuteten Lichtkegeln 26 abgestrahlt, das eine Wellenlänge besitzt, die der halben Wellenlänge der Resonanzfrequenz der Flüssigkeitströpfchen entspricht. Diese Resonanzfrequenz hängt wiederum ab von dem Teilchendurchmesser der jeweiligen Flüssigkeitströpfchen. Die Erfahrung hat gezeigt, daß Licht mit einer Wellenlänge in der Größenordnung von etwa 800 nm recht gut geeignet ist, was einer Teilchengröße der Flüssigkeitströpfchen in der Größenordnung von etwa 1 µm bis 3 µm entspricht.

Die Behandlung der aus dem Zerstäubergefäß 1 austretenden Flüssigkeitströpfchen mit dem Magnetfeld der Magnetspule 8 bzw. der Strahlung von den Strahlungsquellen 17 kann alternativ erfolgen, um den Flüssigkeitströpfchen zusätzliche Energie zuzuführen und diese anzuregen. Besonders gute Ergebnisse werden jedoch dann erzielt, wenn die Behandlung sowohl durch das Magnetfeld der Magnetspule 8 als auch die Bestrahlung mit den Strahlungsquellen 17 erfolgt.

Am Auslaß 14 des Auslaßteiles 15 erkennt man schematisch angedeutete Strahlungsquellen 16 für sichtbares Licht, beispielsweise Licht emittierende Dioden, die sichtbares Licht auf den austretenden Strahl von Flüssigkeitströpfchen abgeben. Damit wird erreicht, daß dieser Flüssigkeitsstrahl sichtbar wird. Damit wird einerseits die Anwendung eines derartigen Strahles von Flüssigkeitströpfchen erleichtert, andererseits hat ein solcher sichtbarer Strahl in einigen Fällen auch eine positive, nämlich beruhigende psychologische Wirkung auf den Benutzer bzw. einen Patienten.

In einigen Fällen kann es erwünscht sein, daß eine weitere Vorbehandlung der Gasströmung erfolgt, bevor diese in das Zerstäubergefäß 1 eingeleitet wird. Zu diesem Zweck ist, wie in Fig. 1 angedeutet, in dem Zuführungsbereich eine Hochspannungselektrode 24, beispielsweise eine Nadelelektrode aus Platin vorgesehen, die an eine Hochspannungsversorgung 23 angeschlossen ist. Auf diese Weise kann beispielsweise eine Korona-Entladung an dieser Platinnadel erzeugt werden, wenn diese auf etwa 6000 V aufgeladen ist.

## Patentansprüche

1. Verfahren zur Erzeugung von Nebeln, bei dem aus einer Flüssigkeit feinstverteilte Flüssigkeitströpfchen erzeugt und anschließend einem zu behandelnden Objekt zugeführt werden, wobei
die Flüssigkeit mit einem Schwingquarz angeregt wird, so daß aus der Flüssigkeit eine aus feinen Flüssigkeitströpfchen bestehende Wolke austritt und
die Flüssigkeitströpfchen mit einer Gasströmung beaufschlagt werden, die eine Strömung von Flüssigkeitströpfchen erzeugt, dadurch gekennzeichnet,
daß die Flüssigkeitströpfchen in der Flüssigkeitströpfchen-Strömung mit einem amplitudenmodulierten Magnetfeld magnetisiert und/oder mit amplitudenmoduliertem Licht bestrahlt werden, bevor sie anschließend zur Behandlung eines Objektes emittiert werden.

2. Verfahren nach Anspruch 1,
dadurch gekennzeichnet,
daß als Flüssigkeit Wasser verwendet wird.

3. Verfahren nach Anspruch 1 oder 2,
dadurch gekennzeichnet,
daß eine Flüssigkeit verwendet wird, die aus einer Trägerflüssigkeit und einer zusätzlichen Wirksubstanz, insbesondere aromatischen Ölen, ätherischen Ölen, homöopathischen Medikamenten, biologischen Substanzen oder Mischungen von diesen besteht.

4. Verfahren nach einem der Ansprüche 1 bis 3,
dadurch gekennzeichnet,
daß die Temperatur der Gasströmung auf einen vorgegebenen Wert eingestellt wird, bevor sie mit den Flüssigkeitströpfchen in Kontakt gebracht wird.

5. Verfahren nach einem der Ansprüche 1 bis 4,
dadurch gekennzeichnet,
daß die Gasströmung ionisiert wird, bevor sie mit den Flüssigkeitströpfchen in Kontakt gebracht wird.

6. Verfahren nach einem der Ansprüche 1 bis 5,
dadurch gekennzeichnet,
daß der Durchsatz bzw. die Geschwindigkeit der zugeführten Gasströmung auf einen vorgegebenen Wert eingestellt wird.

7. Verfahren nach einem der Ansprüche 1 bis 6,
dadurch gekennzeichnet,
daß für die Gasströmung Luft, Sauerstoff, Stickstoff oder ein Inertgas verwendet wird.

8. Verfahren nach einem der Ansprüche 1 bis 7,
dadurch gekennzeichnet,
daß die Flüssigkeit mit einem Schwingquarz bei einer mittleren Resonanzfrequenz von etwa 1,6 MHz angeregt wird.

9. Verfahren nach einem der Ansprüche 1 bis 8,
dadurch gekennzeichnet,
daß das Magnetfeld zur Magnetisierung der Flüssigkeitströpfchen mit einem Rauschspektrum moduliert wird, das in einem Bereich von etwa 1 Hz bis einigen hundert kHz, insbesondere bis 200 kHz liegt.

10. Verfahren nach einem der Ansprüche 1 bis 9,
dadurch gekennzeichnet,
daß die Flüssigkeitströpfchen mit Licht mit einer Wellenlänge bestrahlt werden, die der halben Wellenlänge der Resonanzfrequenz der Flüssigkeitströpfchen entspricht.

11. Verfahren nach einem der Ansprüche 1 bis 10,
dadurch gekennzeichnet,
daß die Flüssigkeitströpfchen mit Licht mit einer Wellenlänge in der Größenordnung von 800 nm bestrahlt werden.

12. Verfahren nach einem der Ansprüche 1 bis 11,
dadurch gekennzeichnet,
daß zur Bestrahlung der Flüssigkeitströpfchen Licht verwendet wird, das mit einem Rauschspektrum amplitudenmoduliert worden ist.

13. Verfahren nach einem der Ansprüche 1 bis 12,
dadurch gekennzeichnet,
daß die Strömung der Flüssigkeitströpfchen bei ihrem Austritt aus der Aufbereitungseinrichtung mit sichtbarem Licht bestrahlt wird.

14. Vorrichtung zur Erzeugung von Nebeln, umfassend
- ein Zerstäubergefäß (1) mit einem Behälter (27) zur Aufnahme einer Flüssigkeit (11),
- eine Einrichtung (20, 21), die der Flüssigkeit (11) Energie zuführt, um aus der Flüssigkeit (11) feinstverteilte Flüssigkeitströpfchen zu erzeugen, und
- ein Gebläse (7), mit dem eine Strömung von Flüssigkeitströpfchen ausgebildet und zum Auslaß (14) der Vorrichtung bewegt wird, von dem die Flüssigkeitströpfchen einem zu behandelnden Objekt zugeführt werden, wobei
der Behälter (27) zur Aufnahme der Flüssigkeit (11) einen Schwingquarz (9) aufweist, der an einen Oszillator (21) angeschlossen ist, dadurch gekennzeichnet,
daß das Zerstäubergefäß (1) einen Kanal (4) aufweist, der von einer Magnetspule (8) umgeben ist, mit der ein amplitudenmoduliertes Magnetfeld erzeugbar ist, und/oder eine Strahlungsquelle (17) besitzt, mit der amplitudenmoduliertes Licht erzeugbar ist, mit dem die Flüssigkeitströpfchen bestrahlt und angeregt werden, bevor sie aus dem Auslaß (14) der Vorrichtung emittiert werden.

15. Vorrichtung nach Anspruch 14,
dadurch gekennzeichnet,
daß der Schwingquarz (9) ein Piezokristall-Wandler, insbesondere ein PXE-Wandler ist, der an einen HF-Generator (21) angeschlossen ist.

16. Vorrichtung nach Anspruch 14 oder 15,
dadurch gekennzeichnet,
daß die Strahlungsquelle (17) mindestens eine Laserdiode aufweist, deren jeweiliger Strahlungskegel (26) die Strömung von Flüssigkeitströpfchen kreuzt.

17. Vorrichtung nach einem der Ansprüche 14 bis 16,
dadurch gekennzeichnet,
daß die Magnetspule (8) an einen Rauschgenerator (20) angeschlossen ist, der ein Rauschspektrum im Bereich von 1 Hz bis einigen hundert kHz, insbesondere bis 200 kHz erzeugt.

18. Vorrichtung nach Anspruch 16 oder 17,
dadurch gekennzeichnet,
daß die Strahlungsquelle (17) an einen Rauschgenerator (19), insbesondere denselben Rauschgenerator (20) wie die Magnetspule (8) angeschlossen ist, der ein Rauschspektrum im Bereich von 1 Hz bis einigen hundert kHz, insbesondere bis 200 kHz erzeugt.

19. Vorrichtung nach einem der Ansprüche 14 bis 18,
dadurch gekennzeichnet,
daß die Strahlungsquelle (19) Licht mit einer Wellenlänge erzeugt, die der halben Wellenlänge der Resonanzfrequenz der Flüssigkeitströpfchen entspricht.

20. Vorrichtung nach einem der Ansprüche 14 bis 19,
dadurch gekennzeichnet,
daß vor dem Auslaß (14) des Kanals (4) ein Homogenisierungsbereich (13) angeordnet ist, der ein drehbares Auslaßteil (15) zur Ausrichtung der Strömung von Flüssigkeitströpfchen aufweist.

21. Vorrichtung nach einem der Ansprüche 14 bis 20,
dadurch gekennzeichnet,
daß der Auslaß (14) mindestens eine weitere Strahlungsquelle (16), insbesondere eine lichtemittierende Diode (LED) aufweist, die sichtbares Licht auf die austretenden Flüssigkeitströpfchen abstrahlt.

22. Vorrichtung nach einem der Ansprüche 14 bis 21,
dadurch gekennzeichnet,
daß das Zerstäubergefäß (1) eine Trenneinrichtung (2) mit einer quer verlaufenden Trennwand (30) und einem hindurchgehenden rohrförmigen Bereich (29) aufweist, in welchem eine Wolke (28) aus Flüssigkeitströpfchen erzeugt wird, die von einem Gasstrom in Bewegung versetzt werden, der unterhalb der Trennwand (20) in das Zerstäubergefäß (1) mündet.

23. Vorrichtung nach Anspruch 22,
dadurch gekennzeichnet,
daß oberhalb des rohrförmigen Bereiches (29) ein plattenförmiger Tropfschutz (3) angeordnet ist, der für große Flüssigkeitstropfen eine Sperre bildet.

24. Vorrichtung nach einem der Ansprüche 14 bis 23,
dadurch gekennzeichnet,
daß das Gebläse (7) an einen einstellbaren Antrieb (22) angeschlossen ist, so daß der Durchsatz bzw. die Austrittsgeschwindigkeit der Gasströmung einstellbar sind.

25. Vorrichtung nach einem der Ansprüche 14 bis 24,
dadurch gekennzeichnet,
daß in dem Zuführungsbereich (5) der Gasströmung des Gebläses (7) eine einstellbare Heizung (6) und/oder Kühlung angeordnet ist.

26. Vorrichtung nach einem der Ansprüche 14 bis 25,
dadurch gekennzeichnet,
daß in dem Zuführungsbereich (5) der Gasströmung des Gebläses (7) eine Ionisierungseinrichtung (24) für das Gas angeordnet ist.

27. Vorrichtung nach einem der Ansprüche 14 bis 26,
dadurch gekennzeichnet,
daß das Zerstäubergefäß (1) an eine Versorgungsquelle (10) für die Flüssigkeit angeschlossen ist, derart, daß die Flüssigkeit in Abhängigkeit vom Flüssigkeitsverbrauch im Zerstäubergefäß (1) automatisch nachfüllbar ist.

28. Vorrichtung nach einem der Ansprüche 14 bis 27,
dadurch gekennzeichnet,
daß der Behälter (27) zur Aufnahme der Flüssigkeit auswechselbar ist.

## Claims

1. Method of producing mists, in which extremely fine liquid droplets are produced from a liquid and subsequently supplied to an object to be treated,
wherein the liquid is agitated with a vibrating quartz so that there emerges from the liquid a cloud of fine liquid droplets and
wherein a stream of gas is caused to impinge on the liquid droplets so as to produce a stream of liquid droplets,
characterized in that
the liquid droplets in the stream of liquid droplets are magnetized with an amplitude-modulated magnetic field and/or with amplitude-modulated light before they are finally emitted for treatment of an object.

2. Method according to Claim 1,
characterized in that water is used as the liquid.

3. Method according to Claim 1 or 2,
characterized in that a liquid is used that consists of a carrier liquid and an additional active substance, in particular aromatic oils, essential oils, homeopathic medications, biological substances or mixtures of thereof.

4. Method according to any of the Claims 1 to 3,
characterized in that the temperature of the gas stream is adjusted to a predetermined value before the gas is brought into contact with the liquid droplets.

5. Method according to any of the Claims 1 to 4,
characterized in that the stream of gas is ionized before it is brought into contact with the liquid droplets.

6. Method according to any of the Claims 1 to 5,
characterized in that the volume flow rate or the velocity of the introduced gas stream is adjusted to a predetermined level.

7. Method according to any of the Claims 1 to 6,
characterized in that air, oxygen, nitrogen or an inert gas is used for the gas stream.

8. Method according to any of the Claims 1 to 7,
characterized in that the liquid is agitated by a vibrating quartz at an average resonant frequency of about 1.6 MHz.

9. Method according to any of the Claims 1 to 8,
characterized in that the magnetic field to magnetize the liquid droplets is modulated with a noise spectrum in a range of about 1 Hz to a few hundred kHz, in particular to 200 kHz.

10. Method according to any of the Claims 1 to 9,
characterized in that the liquid droplets are irradiated with light at a wavelength corresponding to half the wavelength of the resonant frequency of the liquid droplets.

11. Method according to any of the Claims 1 to 10,
characterized in that the liquid droplets are irradiated with light at a wavelength of the order of 800 nm.

12. Method according to any of the Claims 1 to 11,
characterized in that to irradiate the liquid droplets light is used that is amplitude-modulated with a noise spectrum.

13. Method according to any of the Claims 1 to 12,
characterized in that the stream of liquid droplets is irradiated with visible light as it emerges from the preparation apparatus.

14. Apparatus for the production of mists, comprising
- an atomizer vessel (1) with a receptacle (27) to contain a liquid (11),
- a mechanism (20, 21) that introduces energy into the liquid (11) in order to produce extremely fine droplets from the liquid (11), and
- a blower (7) with which to produce a stream of liquid droplets and move it to the outlet (14) of the apparatus, from which the liquid droplets are supplied to an object to be treated,
wherein the receptacle (27) to contain the liquid (11) comprises a vibrating quartz (9) connected to an oscillator (21),
characterized in that
the atomizer vessel (1) comprises a channel (4) surrounded by a magnetic coil (8), with which an amplitude-modulated magnetic field can be generated, and/or comprises a radiation source (17) with which amplitude-modulated light can be generated, with which the liquid droplets are irradiated and excited before they are emitted from the outlet (14) of the apparatus.

15. Apparatus according to Claim 14,
characterized in that the vibrating quartz (9) is a piezoelectric quartz transducer, in particular a PXE transducer, which is connected to an HF generator (21).

16. Apparatus according to Claim 14 or 15,
characterized in that the radiation source (17) comprises at least one laser diode, the radiation cone (26) of which crosses the stream of liquid droplets.

17. Apparatus according to any of the Claims 14 to 16,
characterized in that the magnetic coil (8) is connected to a noise generator (20), which generates a noise spectrum ranging from 1 Hz to a few hundred kHz, in particular to 200 kHz.

18. Apparatus according to Claim 16 or 17,
characterized in that the radiation source (17) is connected to a noise generator (19), in particular to the same noise generator (20) as the magnetic coil (8), which generates a noise spectrum ranging from 1 Hz to a few hundred kHz, in particular to 200 kHz.

19. Apparatus according to any of the Claims 14 to 18,
characterized in that the radiation source (19) generates light with a wavelength corresponding to half the wavelength of the resonant frequency of the liquid droplets.

20. Apparatus according to any of the Claims 14 to 19,
characterized in that there is provided in the channel (4), before its outlet (14), a homogenization region (13) that comprises a rotatable outlet element (15) to direct the stream of liquid droplets.

21. Apparatus according to any of the Claims 14 to 20,
characterized in that the outlet (14) comprises at least one additional radiation source (16), in particular a light-emitting diode (LED), which radiates visible light onto the emerging liquid droplets.

22. Apparatus according to any of the Claims 14 to 21,
characterized in that the atomizer vessel (1) comprises a partitioning device (2) with a transversely oriented partition (30) through which passes a tubular region (29) in which is produced a cloud (28) of liquid droplets, which are set in motion by a stream of gas that flows into the atomizer vessel (1) below the partition (30).

23. Apparatus according to Claim 22,
characterized in that above the tubular region (29) there is disposed a plate-like drop baffle (3), which constitutes a barrier to large drops of liquid.

24. Apparatus according to any of the Claims 14 to 23,
characterized in that the blower (7) is connected to an adjustable drive mechanism (22) so that the volume flow rate and/or the exit velocity of the stream of gas can be adjusted.

25. Apparatus according to any of the Claims 14 to 24,
characterized in that in the entrance region (5) of the stream of gas propelled by the blower (7) there is disposed an adjustable heating device (6) and/or cooling device.

26. Apparatus according to any of the Claims 14 to 25,
characterized in that in the entrance region (5) of the stream of gas propelled by the blower (7) there is disposed an ionizing device (24) for the gas.

27. Apparatus according to any of the Claims 14 to 26,
characterized in that the atomizer vessel (1) is connected to a reservoir (10) of the liquid, in such a way that the liquid in the atomizer vessel (1) is automatically replenished in dependence on its rate of consumption.

28. Apparatus according to any of the Claims 14 to 27,
characterized in that the receptacle (27) to contain the liquid is exchangeable.

## Revendications

1. Procédé pour la production de brouillard, dans lequel on produit à partir d'un liquide des gouttelettes de liquide finement réparties, et on les amène ensuite à un objet à traiter,
dans lequel le liquide est excité avec un quartz oscillant de sorte qu'il sort du liquide un nuage constitué de fines gouttelettes de liquide, et
les gouttelettes de liquide sont attaquées par un courant de gaz qui produit un écoulement de gouttelettes de liquide,
caractérisé en ce que
les gouttelettes de liquide dans l'écoulement de gouttelettes de liquide soit aimantées avec un champ magnétique modulé en amplitude et/ou irradiées par une lumière modulée en amplitude, avant d'être ensuite émises pour le traitement d'un objet.

2. Procédé selon la revendication 1,
caractérisé en ce que l'on utilise de l'eau en tant que liquide.

3. Procédé selon l'une ou l'autre des revendications 1 et 2,
caractérisé en ce que l'on utilise un liquide qui est constitué par un liquide porteur et par une substance active additionnelle, en particulier des huiles aromatiques, des huiles essentielles, des médicaments homéopathiques, des substances biologiques, ou des mélanges de ceux-ci.

4. Procédé selon l'une des revendications 1 à 3,
caractérisé en ce que la température de l'écoulement de gaz est réglée à une valeur prédéterminée avant d'être amené en contact avec les gouttelettes de liquide.

5. Procédé selon l'une des revendications 1 à 4,
caractérisé en ce que l'écoulement de gaz est ionisé avant d'être amené en contact avec les gouttelettes de liquide.

6. Procédé selon l'une des revendications 1 à 5,
caractérisé en ce que le débit ou la vitesse de l'écoulement de gaz amené est réglé(e) à une valeur prédéterminée.

7. Procédé selon l'une des revendications 1 à 6,
caractérisé en ce que l'on utilise pour l'écoulement de gaz de l'air, de l'oxygène, de l'azote ou un gaz inerte.

8. Procédé selon l'une des revendications 1 à 7,
caractérisé en ce que le liquide est excité avec un quartz oscillant à une fréquence de résonance moyenne d'environ 1,6 MHz.

9. Procédé selon l'une des revendications 1 à 8,
caractérisé en ce que le champ magnétique pour aimanter les gouttelettes de liquide est modulé avec un spectre de bruit situé dans une plage depuis environ 1 Hz jusqu'à quelques centaines de kHz, en particulier jusqu'à 200 kHz.

10. Procédé selon l'une des revendications 1 à 9,
caractérisé en ce que les gouttelettes de liquide sont irradiées par de la lumière avec une longueur d'onde qui correspond à la demi longueur d'onde de la fréquence de résonance des gouttelettes de liquide.

11. Procédé selon l'une des revendications 1 à 10,
caractérisé en ce que les gouttelettes de liquide sont irradiées par de la lumière avec une longueur d'onde de l'ordre de grandeur de 800 nm.

12. Procédé selon l'une des revendications 1 à 11,
caractérisé en ce que pour irradier les gouttelettes de liquide on utilise une lumière qui a été modulée en amplitude avec un spectre de bruit.

13. Procédé selon l'une des revendications 1 à 12,
caractérisé en ce que l'écoulement des gouttelettes de liquide est irradié avec de la lumière visible à sa sortie hors du dispositif de préparation.

14. Appareil pour la production de brouillard, comprenant :
- un récipient de pulvérisation (1) avec un réservoir (27) pour recevoir un liquide (11),
- un dispositif (20, 21) qui amène au liquide (11) de l'énergie, afin de produire à partir du liquide (11) des gouttelettes de liquide finement réparties, et
- un ventilateur (7), avec lequel on forme un écoulement de gouttelettes de liquide et on le déplace vers la sortie (14) de l'appareil, depuis laquelle les gouttelettes de liquide sont amenées à un objet à traiter,
dans lequel le réservoir (27) pour la réception de liquide (11) comporte un quartz oscillant (29), auquel est raccordé un oscillateur (21),
caractérisé en ce que
le récipient de pulvérisation (1) comporte un canal (4) qui est entouré par un bobinage magnétique (8) au moyen duquel on peut produire un champ magnétique modulé en amplitude, et/ou comporte une source de rayonnement (17) au moyen de laquelle on peut produire une lumière modulée en amplitude, avec laquelle les gouttelettes de liquide sont irradiées et excitées avant d'être émises hors de la sortie (14) de l'appareil.

15. Appareil selon la revendication 14,
caractérisé en ce que le quartz oscillant (9) est un convertisseur à piézocristal, en particulier un convertisseur PXE, qui est raccordé à un générateur HF (21).

16. Appareil selon l'une ou l'autre
des revendications 14 et 15,
caractérisé en ce que la source de rayonnement (17) comprend au moins une diode laser, dont les cônes de rayonnement respectifs (26) croisent l'écoulement des gouttelettes de liquide.

17. Appareil selon l'une des revendications 14 à 16,
caractérisé en ce que le bobinage magnétique (8) est raccordé à un générateur de bruit (20), lequel produit un spectre de bruit dans la plage allant depuis 1 Hz jusqu'à quelques centaines de kHz, en particulier jusqu'à 200 kHz.

18. Appareil selon l'une ou l'autre
des revendications 16 et 17,
caractérisé en ce que la source de rayonnement (17) est raccordée à un générateur de bruit (19), en particulier le même générateur de bruit (20) que le bobinage magnétique (8), qui produit un spectre de bruit dans la plage depuis 1 Hz jusqu'à quelques centaines de kHz, en particulier jusqu'à 200 kHz.

19. Appareil selon l'une des revendications 14 à 18,
caractérisé en ce que la source de rayonnement (19) produit de la lumière avec une longueur d'onde qui correspond à la demi longueur d'onde de la fréquence de résonance des gouttelettes de liquide.

20. Appareil selon l'une des revendications 14 à 19,
caractérisé en ce que, avant la sortie (14) du canal (4), est agencée une zone d'homogénéisation (13), qui présente une partie de sortie tournante (15) pour diriger l'écoulement de gouttelettes de liquide.

21. Appareil selon l'une des revendications 14 à 20,
caractérisé en ce que la sortie (14) comprend au moins une autre source de rayonnement (16), en particulier une diode électroluminescente (LED), qui émet de la lumière visible sur les gouttelettes de liquide sortantes.

22. Appareil selon l'une des revendications 14 à 21,
caractérisé en ce que le récipient de pulvérisation (1) comprend un dispositif de séparation (2) avec une cloison transversale (30) et une zone tubulaire traversante (29), dans laquelle est produit un nuage (28) de gouttelettes de liquide, lesquelles sont mises en mouvement par un écoulement gaz, qui débouche dans le récipient de pulvérisation (1) au-dessous de la cloison (30).

23. Appareil selon la revendication 22,
caractérisé en ce qu'une protection anti-gouttes (3) en forme de plaque est agencée au-dessus de la zone tubulaire (29), et forme un blocage pour les gouttelettes de liquide de grande taille.

24. Appareil selon l'une des revendications 14 à 23,
caractérisé en ce que le ventilateur (7) est raccordé à un entraînement réglable (22) de sorte que l'on peut régler le débit ou la vitesse de sortie de l'écoulement de gaz.

25. Appareil selon l'une des revendications 14 à 24,
caractérisé en ce dans une région d'admission (5) de l'écoulement de gaz dans le ventilateur (7) est agencé un chauffage (6) et/ou un refroidissement réglable.

26. Appareil selon l'une des revendications 14 à 25,
caractérisé en ce que dans la région d'admission (5) de l'écoulement de gaz dans le ventilateur (7) est agencé un dispositif d'ionisation (24) pour le gaz.

27. Appareil selon l'une des revendications 14 à 26,
caractérisé en ce que le récipient de pulvérisation (1) est raccordé à une source d'alimentation (10) pour le liquide, de telle sorte que le liquide est complété automatiquement en fonction de la consommation de liquide dans le récipient de pulvérisation (1).

28. Appareil selon l'une des revendications 14 à 27,
caractérisé en ce que le réservoir (27) pour recevoir le liquide est interchangeable.
